# EUROPEAN PATENT SPECIFICATION

(11) **EP 0 305 174 B1**
(45) Date of publication and mention of the grant of the patent: **15.09.1993**
(21) Application number: 88307861.0
(22) Date of filing: 24.08.1988
(51) Int. Cl.: A61K 37/02

(54) **Peptide FK-565 useful for controlling helminthic parasites**
Peptid FK-565, brauchbar zur Kontrolle helminthischer Parasiten
Peptide FK-565 utile pour le contrôle des parasites helminthiques

(30) Priority: 24.08.1987 US 88818
(43) Date of publication of application: 01.03.1989
(73) Proprietor: PITMAN-MOORE, INC., Terre Haute Indiana 47808 (US)
(72) Inventor: Clough, Ellen R., Illinois 60126 (US); Edwards, Carl K. III, Terre Haute, Indiana 47802 (US)
(74) Representative: Holmes, Michael John

(56) References cited:
- US-A- 4 322 341
- CANCER IMMUNOLOGY IMMUNOTHERAPY, vol.18, 18th March 1984, pages 169-173, Springer-Verlag; S. SONE et al.: "Activation by a new synthetic acyltripeptide and its analogs entrapped in liposomes of rat alveolar macrophages to the tumor cytotoxic state"
- THE JOURNAL OF ANTIBIOTICS, vol. 36, no.8, August 1983, pages 1045-1050; Y. MINE et al.: "Immunoactive peptides, FK-156 and FK-565. I enhancement of host resistance to microbial infection in mice."

## Description

This invention relates generally to the control of helmintic parasites and particularly to the use of the known peptide FK-565 for the preparation of compositions for controlling helmintic parasites.

Diseases caused by helmintic parasite infections far outnumber diseases caused by other infectious agents. It is estimated that one billion people are infected with roundworms alone and that eight hundred million people are infected with hookworm. This does not include infections caused by other helmintic parasites nor include the millions and perhaps billions of animals infected with helmintic parasites. The taxonomic, identification, classification, and history of helmintic parasites and the epidemiology, treatment, and conduct of the more prevalent parasitic infections is reviewed in Parasitology for Veterinarians by J.R. Georgi, W.B. Sanders Co., 3rd edition (1980).

Attempts to control helmintic parasites are disclosed in ancient Chinese and Egyptian writings dating back 3500 years. More recently, there has been a continuing effort to develop methods for treating infected animals and controlling the spread of helmintic parasite infections. Broad spectrum anthelmintic agents such as aminoglycoside antibodies, organophosphorous compounds, benzimidazoles, organic arsenic compounds, piparazines, imidoylureas are a few among many anthelmintic agents that have been used to treat and control the spread of helmintic parasite infections. These anthelmintic agents generally function by destroying helmintic parasites in various developmental stages including adults, larvae, and eggs. Many of these compounds, however, are toxic to the host in effective dosages, difficult to prepare or synthesize, expensive, or produce adverse side effects when administered to the host animal. There exists, therefore, a continuing need for new and more effective methods for controlling helmintic parasites.

Previous methods for controlling helmintic parasites include the following: U.S. Patent No. 3,390,148 discloses the use of thioimidates as anthelmintic agents. U.S. Patent No. 3,458,633 discloses the use of thiazoline derivatives as anthelmintic agents. U.S. Patent No. 3,915,986 discloses the use of 5-propylthio-2-benzimidazole carbamate against gastro-intestinal parasites in animals. U.S. Patent No. 3,721,740 discloses the use of phenylhydrazone derivatives as anthelmintic agents. U.S. Patent No. 4,175,135 discloses a method for controlling acarina ectoparasites using 2-aryl-1,3-cyclohexanedione. U.S. Patent No. 4,255,447 discloses the use of phenylcyclopropane carboxylic acid derivatives having acaracidal properties. U.S. Patent No. 4,299,837 discloses using benzimidazole-carbamates as anthelmintic agents. U.S. Patent No. 4,468,390 discloses anthelmintic compositions comprising antibiotics, benzimidazole, salicylamide, and isoquinoline compounds. U.S. Patent No. 4,105,779 discloses oral anthelmintic compositions comprising phosphate compounds and thermoplastic resins. U.S. Patent No. 4,287,176 discloses using thermally revisable gels containing levamisole, tetramisole, butamisole and benzamisole. U.S. Patent No. 4,348,389 discloses administering substituted quinoxaline adducts to infested animals to control parasitic worms. U.S. Patent No. 4,337,274 discloses flukicidal diphenylether compounds. U.S. Patent No. 4,593,024 to Lu et al. discloses dihydroisoxazole compounds having anthelmintic properties. U.S. Patent No. 4,009,266 discloses a method for controlling gastro-intestinal nematode parasites in domestic animals. U.S. Patent No. 3,978,060 discloses new compounds used to eradicate internal parasites. U.S. Patent No. 3,980,791 discloses pour-on tetramisole and levamisole anthelmintic compositions.

The peptide, FK-565, of the present invention is a known compound. FK-565 is a macrophage activator which is reported to increase host resistance to infection, induce interleukin-1 production in macrophages, and enhance phagocytic and killing activities of macrophages. Agric. Biol. Chem., 48(9):2393-2394 (1984). FK-565 is a synthetic derivative of a natural peptide FK-156 isolated from the culture filtrate of strains of Streptomyces olivaceogriseus sp nov. and Streptomyces violaceus. The isolation, purification, and structure of FK-156 has been thoroughly reviewed in the literature: J.Antibiotics, 35:1280-85 (1982) (Taxonomy of the Producing Strains); J.Antibiotics, 35:1286-92 (1982) (Fermentation, Extraction and Chemical and Biological Characterization); J.Antibiotics, 35:1293-99 (1982) (Structure Elucidation); and J.Antibiotics, 35:1300-11 (1982) (Synthesis of FK-156 and Its Geometric Isomer). U.S. Patent No. 4,311,640 to Kuroda et al. discloses methods for preparing FK-156 and some of its derivatives. U.S. Patent No. 4,322,341 to Kitaura et al. discloses methods for producing FK-565. U.S. Patent No. 4,349,466 to Kitaura et al. discloses methods for producing several peptides related to FK-565.

FK-156 has the structure: 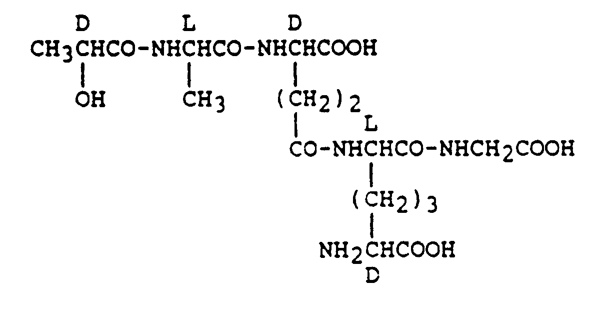
FK-565 has the structure: 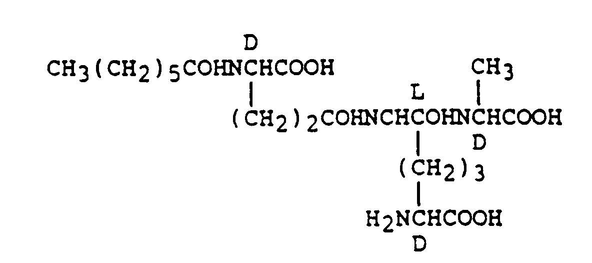
The present invention seeks to solve the problem of treating and/or controlling helmintic parasites in animals or humans.

Thus, in one aspect, the invention provides use of FK-565 for the preparation of a composition for use in the treatment and/or prevention of helmintic parasite infections in an animal.

These and other objects are achieved by administering helmintic parasite controlling amounts of the peptide FK-565 to the animal. FK-565 is administered in dosages from 0.001-5 mg/kg of body weight during or prior to an anticipated helmintic parasite infection to prevent or treat the disease and the resulting clinical manifestations. Preferably, FK-565 is administered to the animal prior to an anticipated infection to prevent the resulting disease or administered to the animal after the disease occurs to reduce the parasite population.

Preferably, FK-565 is used for administeration to animals infected by trematodes, cestodes, and nematodes, particularly migratory respiratory and gastro-intestinal parasites, in dosages from 0.1-2.5 mg/kg of body weight to control the parasite population.

FK-565 is used in admixture with a pharmaceutically acceptable carrier to form a composition useful for controlling helmintic parasites. The composition is administered to animals or humans infected with or susceptible to infection by helmintic parasites to control the parasite population.

Other objects, advantages, and novel features of the present invention will become apparent from the following detailed description of the invention.

The following terms used herein are defined as follows: "helmintic parasite" is defined to include parasites in the adult, larvae, and egg stage; and "to control" and "controlling" helmintic parasites are defined to include (1) administering FK-565 to helmintic parasite infected animals or humans to treat the disease by eliminating or reducing the helmintic parasite population, and (2) administering FK-565 to noninfected animals or humans to prevent future infection by helmintic parasites. FK-565 is defined to include not only the peptide but also its biologically active and pharmaceutically acceptable salts and esters.

A method for controlling helmintic parasites in animals or humans comprises administering FK-565 to the animal or human, preferably by administering a composition for controlling helmintic parasites in animals or humans comprising FK-565 used in admixture with an inert pharmaceutical carrier. The compound or the composition is administered to animals or humans infected with or susceptible to infection by helmintic parasites to control the parasite population.

The present invention can be used to control a broad range of helmintic parasites including but not limited to trematodes, cestodes, and nematodes, particularly flukes, lungworms, roundworms, tapeworms, pinworms, and hookworms. In the preferred embodiment, FK-565 is administered to animals or humans to control respiratory and gastro-intestinal cestodes and nematodes, particularly migratory parasites such as lungworms, roundworms and tapeworms.

Any animal species or human susceptible to infection by helmintic parasite can be administered FK-565. Human, bovine, porcine, canine, feline, equine, avian, and ovine are preferred, with livestock and poultry such as cattle, swine, sheep, chickens, and turkeys being most preferred.

Although the amount of FK-565 administered to the animals or humans can vary depending on the type of animal, type of infection, degree of infection, and the like, typical dosages administered to the animal or human range from 0.001-5 mg/kg of body weight. Preferably, animals or humans infected with helmintic parasites are administered from 0.1-2.5 mg/kg of body weight to combat the infection. Animals or humans susceptible to infection by helmintic parasites are administered from 0.1-2.5 mg/kg of body weight to prevent an infection.

FK-565 can be administered to the animals or humans in any acceptable manner including orally, by injection and using an implant. Injections and implants permit precise control of the timing and dosage levels used for administration while oral administration is more convenient. FK-565 can also be administered parenterally. As used herein, parenteral administration means administration by intravenous, intramuscular, subcutaneous, or intraperitoneal injection, or by subcutaneous implant.

FK-565 can be administered orally to the animal or human. Oral administration includes administering FK-565 in tablets, suspensions, implants, solutions, boluses, emulsions, capsules, powders, syrups, water compositions and feed compositions. For example, FK-565 can be blended with ordinary feed compositions or added to drinking water in amounts sufficient to treat or prevent helmintic parasite infections.

When FK-565 is to be administered in feeds, an animal feed composition may be prepared containing the usual nutritionally-balanced feed containing quantities of carbohydrates, proteins, vitamins and minerals, together with FK-565 in accordance with the present invention. Some of the usual dietary elements included in animal feed compositions are grains, such as ground grain and grain byproducts, animal protein substances, such as those found in fish meal and meat scraps, vegetable proteins, like soybean oil meal or peanut oil meal; vitamins and vitamin-containing materials, e.g., vitamin A and D mixtures, riboflavin supplements and other vitamin B complex members; and bone meal and limestone to provide minerals. A type of conventional feed material for use with cattle includes alfalfa hay and ground corncobs together with supplementary vitamins and vitamin-containing substances if desired. Similarly, a medicated animal feed composition based on liver meal, such as disclosed in U.S. Pat. No. 4,283,400 can be used to administer FK-565.

FK-565 is used in admixture with the feed in amounts sufficient to supply from 0.001-5 mg/kg of body weight, typically 11-110mg/Kg (10-100 grams/ton) of feed, to the animal.

FK-565 can be administered to the animals or humans in an injectable formulation containing any biocompatible and FK-565 compatible carrier such as various vehicles, adjuvants, additives, and diluents. Such formulations and carriers are well known in the art. FK-565 is added to the carrier in amounts sufficient to supply from 0.0 01-5 mg/kg of body weight to the animals when injected. Preferably, FK-565 is added to the carrier in amounts sufficient to supply from 0.1-2.5 mg/kg of body weight to prevent infection.

Aqueous vehicles such as water having no nonvolatile pyrogens, sterile water, and bacteriostatic water are also suitable to form injectable FK-565 formulations. In addition to these forms of water, several other aqueous vehicles can be used. These include isotonic injection compositions that can be sterilized such as sodium chloride, carboxymethylcellulose (CMC), Ringer's, dextrose, dextrose and sodium chloride, and lactated Ringer's. Addition of water-miscible solvents, such as methanol, ethanol, or propylene glycol generally increases solubility and stability of FK-565 in these vehicles.

Nonaqueous vehicles such as cottonseed oil, sesame oil, or peanut oil and esters such as isopropyl myristate may also be used as solvent systems for the FK-565 compositions. Additionally various additives which enhance the stability, sterility, and isotonicity of the composition including antimicrobial preservatives, antioxidants, chelating agents, and buffers can be added. Any vehicle, diluent, or additive used would, however, have to be compatible with FK-565. Preferably, FK-565 is administered in a squalene, HBSS, physiologic saline, or CMC vehicle.

FK-565 can be administered to the animals or humans in the form of a slow-release subcutaneous implant which is inserted beneath the skin of the animals or humans. The implant can take the form of a pellet which slowly dissolves after being implanted in the animals or a biocompatible and FK-565 compatible delivery module well known to those skilled in the art. Such well known dosage forms are designed such that the active ingredients are slowly released over a period of several days to several weeks.

The implant according to the present invention, is designed to deliver FK-565 in amounts from 0.001-5 mg/kg body weight/day, preferably from 0.1-2.5 mg/kg body weight/day.

The invention having been generally described, the following examples are given as particular embodiments of the invention and to demonstrate the practice and advantages thereof.

### EXAMPLE 1

The anthelmintic activity of FK-565 was evaluated against the parasitic nematode, Nippostrongylus brasiliensis, in experimentally infected CF₁ male mice. Forty (40) mice, 4-6 weeks of age weighing approximately 18-20 grams each, were acclimated to new cages and fed pelleted rodent chow (Purina #5002) and water ad libitum. The mice were divided into 8 treatment groups; each treatment group consisted of 5 mice per cage with no replicates. Each treatment group was administered FK-565 in 1% squalene or a control as follows:

| Group | Treatment | Vehicle |
|---|---|---|
| 1 | 25 µg FK-565 | 1% squalene |
| 2 | 5 µg FK-565 | 1% squalene |
| 3 | 1 µg FK-565 | 1% squalene |
| 4 | 0.1 µg FK-565 | 1% squalene |
| 5 | 0.01 µg FK-565 | 1% squalene |
| 6 | 1% squalene | HBSS |
| 7 | Levamisole | |
| 8 | Non-treated infected | |

The FK-565 compositions and controls were administered via subcutaneous injection. Groups 1-5 were treated with an emulsion of the experimental compound in 1% squalene. Group 6 was treated with 1% squalene in HBSS (Hank's Balanced Salt Solution) while Group 7 was treated with Levamisole, a commercial anthelmintic product, administered at a dosage. of 9.6 µg/mouse and serving as a positive control. Group 8 served as the negative control with infections with the parasite but no treatments administered.

Twenty-four (24) hours after treatments were administered, individual mice were injected subcutaneously with a single dose (0.1 ml) of the infective stage Nippostrongylus brasiliensis larvae (approximately 300 L3 forms per injection). The actual number of larvae were counted prior to injection.

The mice were continued on water and feed for seven days and were observed for any changes in physical well being.

On the seventh day after injection, the mice were taken off feed in the evening and fasted overnight. The mice were sacrificed the next day and the small intestine of each mouse removed. The small intestine was compressed between two heavy glass plates and the red, adult worms counted. To determine efficacy, the number of worms recovered in drug treated mice were compared with those of non-medicated control mice. The number of adult worms recovered from animals treated intravenously, one day after Nippostrongylus infection, with 0.01, 0.1, 1, 5, or 25 µg FK-565 in the 1% squalene in HBSS emulsion was determined and compared with the negative control, animals treated with the emulsion only. The results are shown in Table 1.

Referring to Table 1, approximately 229 adult worms were recovered from untreated mice challenged with 300 L3 larvae. The value, 229, was used to determine the percent worm reduction in treated animals. Administration of 25 µg/mouse FK-565 reduced the worm burden by 60%. Groups treated with either 5, 1, or .01 µg/mouse exhibited a 47% reduction in adult worms. Treatment with 0.1 µg/mouse resulted in only a 21% reduction. Since the standard deviation within this group is only 7, the percent efficacy is very similar to that observed when animals were treated with the squalene in HBSS emulsion alone (19%), it is believed that these animals were mistakenly never treated with FK-565.

Thus mice given FK-565 in the squalene vehicle were protected in a dose-related fashion against the migrating larvae through the lungs. There was minimal protection with the vehicle alone (19% efficacy).

### EXAMPLE 2

The anthelmintic activity of FK-565 was evaluated against the parasitic nematode, Nippostrongylus brasiliensis, in experimentally infected CF₁ male mice. Sixty (60) mice, 4-6 weeks of age weighing approximately 18-20 grams each, were acclimated to new cages and fed pelleted rodent chow (Purina #5002) and water ad libitum. The mice were divided into 12 treatment groups; each treatment group consisted of 5 mice per cage with no replicates. The treatment groups are as follows:

| Group | Treatment | Vehicle | Route |
|---|---|---|---|
| 1 | 100 µg FK-565 | 1% squalene/HBSS | s.c. |
| 2 | 50 µg FK-565 | 1% squalene/HBSS | s.c. |
| 3 | 25 µg FK-565 | 1% squalene/HBSS | s.c. |
| 4 | 5 µg FK-565 | 1% squalene/HBSS | s.c. |
| 5 | 1 µg FK-565 | 1% squalene/HBSS | s.c. |
| 6 | - | 1% squalene/HBSS | s.c. |
| 7 | 25 µg FK-565 | HBSS | s.c. |
| 8 | 5 µg FK-565 | HBSS | s.c. |
| 9 | 25 µg FK-565 | 1% squalene/HBSS | i.v. |
| 10 | 5 µg FK-565 | 1% squalene/HBSS | i.v. |
| 11 | - | 1% squalene/HBSS | i.v. |
| 12 (untreated,infected) | | - | -- |
| s.c. = subcutaneously; i.v. = intravenously | | | |

Twenty-four (24) hours before treatments were administered, individual mice were injected subcutaneously with a single dose (0.1 ml) of the infective stage Nippostrongylus brasiliensis larvae (approximately 300 L3 forms per injection). The actual number of larvae were counted prior to injection.

The mice were continued on water and feed for seven days and were observed for any changes in physical well being.

On the seventh day after infection, the mice were taken off feed in the evening and fasted overnight. The following day the mice were sacrificed and the small intestine of each mouse was removed. The small intestine was compressed between two heavy glass plates and the red, adult worms counted. To determine efficacy, the number of worms recovered in drug-treated mice were compared with those in non-medicated control mice. The results are shown in Table 2.

Referring to Table 2, the data shows a dose-dependent reduction of adult, mature worms in the gut of mice previously treated with FK-565, given subcutaneously in a 1% squalene/HBSS emulsion. The mice treated with 25-100 µg FK-565 showed the greatest reduction. The range of efficacy was found to be 45% to 71%. The vehicle, 1% squalene in HBSS alone, given either s.c. or i.v., afforded minimal protection. The efficacy of subcutaneous delivery of FK-565 in oil was compared with subcutaneous delivery in HBSS and with intravenous delivery in the emulsion at two doses of FK-565, 5 and 25 µg/mouse. The results show that the three treatments were comparable in their ability to reduce worm burdens.

The data in Table 2 shows that FK-565, when given at the appropriate dose and route, has anthelmintic activity. Although the exact mechanism of how FK-565 may be working remains to be elucidated, it is clear that this compound, delivered s.c. at dosages ranging from 0.01 µg/mouse to 100 µg/mouse, reduces adult worm burdens in mice infected with N. brasiliensis. The data also shows that FK-565 is active regardless of the vehicle into which it is suspended, and regardless of the route by which it is given.

### EXAMPLE 3

The anthelmintic activity of FK-565 was evaluated against the parasitic nematode, Nippostrongylus brasiliensis, in experimentally infected CF₁ male mice, to determine if the time of treatment with respect to challenge with Nippostrongylus larvae has an effect on the magnitude of reduction of adult worms recovered from the intestine. One hundred fifty (150) mice, 4-6 weeks of age weighing approximately 18-20 grams each, were acclimated to new cages and fed pelleted rodent chow (Purina #5002) and water ad libitum. The mice were divided into treatment groups; each treatment group consisted of 5 mice per cage with no replicates. Each treatment group was administered FK-565 in various vehicles or a control as follows:

| Treatment | Day |
|---|---|
| 50 µg FK-565 (oral) | -6 |
| 25 µg FK-565 (s.c.) | -6 |
| HBSS | -6 |
| 50 µg FK-565 (oral) | -3 |
| 25 µg FK-565 (s.c.) | -3 |
| HBSS | -3 |
| 50 µg FK-565 (oral) | -1 |
| 25 µg FK-565 (s.c.) | -1 |
| HBSS | -1 |
| 50 µg FK-565 (oral) | +1 |
| 25 µg FK-565 (s.c.) | +1 |
| HBSS | +1 |
| 50 µg FK-565 (oral) | +3 |
| 25 µg FK-565 (s.c.) | +3 |
| HBSS | +3 |
| 50 µg FK-565 (oral) | +6 |
| 25 µg FK-565 (s.c.) | +6 |
| HBSS | +6 |

Treatments were given on days -6, -3, -1, +1, +3, or +6 before or after subcutaneous infection with Nippostrongylus brasiliensis L3 infective larvae. On day 0, all mice were injected subcutaneously with a single dose (0.1 ml) containing approximately 300 infective stage larvae.

The mice were continued on water and feed for seven days and were observed for any changes in physical well being.

On the seventh day after infection, the mice were taken off feed in the evening and fasted overnight. The following day the mice were sacrificed and the small intestine of each mouse removed. The small intestine was compressed between two heavy glass plates and the red, adult worms counted. To determine efficacy, the number of worms recovered in drug-treated mice were compared with those in non-medicated control mice. The results are shown in Table 3.

Referring to Table 3, treatment with FK-565 on day -1 reduces the number of adult worms recovered by approximately 57%. Treatment on days -6, -3, +1, +3, or +6 also resulted in decreases in worm recovery. Treatment on day -1 with 50 µg FK-565 given orally resulted in a reduction of 64%. This indicates that FK-565 is capable of acting as an anthelmintic when given orally.

Obviously many modifications and variations of the present invention are possible in light of the above teachings. It is therefore to be understood that within the scope of the appended claims the invention may be practiced otherwise than as specifically described.

**TABLE 1**

| Treatment* | Average Worms Recovered | % Efficacy |
|---|---|---|
| 25 µg FK-565 | 91 ± 4 | 60 |
| 5 µg FK-565 | 121 ± 7 | 47 |
| 1 µg FK-565 | 121 ± 6 | 47 |
| 0.1 µg FK-565 | 180 ± 7 | 21 |
| 0.01 µg FK-565 | 122 ± 18 | 47 |
| Squalene (1% in HBSS) | 185 ± 9 | 19 |
| Levamisole | 0 | 100 |
| Non-Treated Infected | 229 ± 7 | 0 |

| | | |
|---|---|---|
| *All treatments were in 1% squalene in HBSS emulsion and were delivered i.v. | | |

**TABLE 2**

| Treatment* | Average Worms Recovered | % Efficacy |
|---|---|---|
| 100 µg | 65 ± 8 | 71 |
| 50 µg | 78 ± 7 | 65 |
| 25 µg | 88 ± 7 | 60 |
| 5 µg | 112 ± 6 | 49 |
| 1 µg | 121 ± 8 | 45 |
| Squalene (1%/HBSS) | 197 ± 8 | 11 |
| 25 µg FK-565 in HBSS | 87 ± 6 | 61 |
| 5 µg FK-565 in HBSS | 108 ± 9 | 51 |
| 25 µg (i.v.) | 82 ± 6 | 63 |
| 5 µg (i.v.) | 97 ± 11 | 56 |
| Squalene (1%/HBSS,i.v.) | 192 ± 4 | 13 |
| Untreated, Infected | 221 ± 7 | 0 |

| | | |
|---|---|---|
| *All treatments given s.c. in 1% squalene in HBSS unless noted. | | |

**TABLE 3**

| Treatment | Day | Worms | %Efficacy |
|---|---|---|---|
| 50 µg FK-565 (oral) | -6 | 105 ± 7 | 46 |
| 25 µg FK-565 (s.c.) | -6 | 117 ± 8 | 41 |
| HBSS | -6 | 205 ± 7 | |
| 50 µg FK-565 (oral) | -3 | 97 ± 8 | 52 |
| 25 µg FK-565 (s.c.) | -3 | 95 ± 9 | 53 |
| HBSS | -3 | 202 ± 7 | |
| 50 µg FK-565 (oral) | -1 | 72 ± 6 | 64 |
| 25 µg FK-565 (s.c.) | -1 | 87 ± 7 | 57 |
| HBSS | -1 | 204 ± 12 | |
| 50 µg FK-565 (oral) | +1 | 89 ± 6 | 56 |
| 25 µg FK-565 (s.c.) | +1 | 91 ± 3 | 55 |
| HBSS | +1 | 203 ± 8 | |
| 50 µg FK-565 (oral) | +3 | 89 ± 4 | 55 |
| 25 µg FK-565 (s.c.) | +3 | 106 ± 10 | 47 |
| HBSS | +3 | 199 ± 8 | |
| 50 µg FK-565 (oral) | +6 | 106 ± 5 | 50 |
| 25 µg FK-565 (s.c.) | +6 | 111 ± 6 | 47 |
| HBSS | +6 | 210 ± 6 | |

## Claims

1. Use of the peptide FK-565 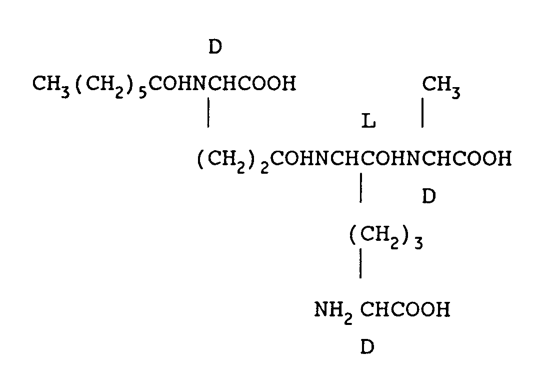 for the preparation of a composition for use in the treatment and/or control of helmintic parasite infections in an animal or human.

2. Use as claimed in claim 1 wherein said helmintic parasites are selected from trematodes, cestodes, and nematodes.

3. Use as claimed in claim 1 or claim 2 wherein said helmintic parasites are selected from helmintic parasites that infect said animal's respiratory and gastro-intestinal tract.

4. Use as claimed in claim 1 wherein said helmintic parasites are selected from flukes, lungworms, roundworms, tapeworms, pinworms, and hookworms.

5. Use as claimed in any one of claims 1 to 4 wherein said animals are selected from bovine, porcine, canine, feline, equine, avian, and ovine.

6. Use as claimed in claim 5 wherein the animals are selected from cattle, swine, sheep, chickens and turkeys.

7. Use as claimed in any one of claims 1 to 6 wherein said composition comprises said peptide FK- 565 in an amount sufficient to supply from 0.001 to 5 mg/kg body weight to said animal or human.

8. Use as claimed in any one of claims 1 to 7 for the preparation of a composition in the form of an injectable formulation suitable for controlling helmintic parasites in animals or humans.

9. Use as claimed in claim 8 wherein said injectable formulation comprises a vehicle selected from squalene, carboxymethylcellulose (CMC), physiologic saline, or Hank's Balanced Salt Solution.

10. Use as claimed in any one of claims 1 to 7 for the preparation of a composition in a form suitable for oral administration.

11. Use as claimed in claim 10 for the preparation of a composition in the form of tablets, suspensions, solutions, boluses, emulsions, capsules, powders, syrups, drinking water compositions, and feed compositions.

12. A feed composition for oral administration suitable for controlling helmintic parasites in an animal or human, in the form of a feed composition comprising:
a nutritionally balanced feed; and
a helmintic parasite controlling amount of the peptide FK-565 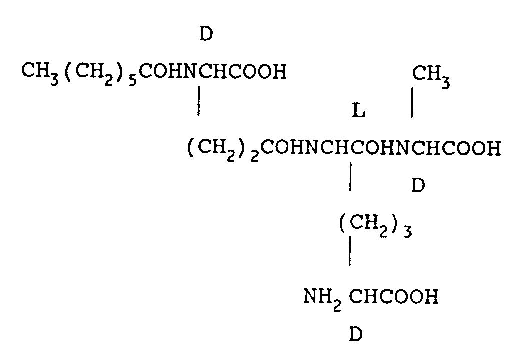 admixed with said feed.

13. The feed composition of claim 12 wherein said FK-565 is applied to or used in admixture with said feed composition in amounts of from 11 to 110 mg/kg (10 to 100 grams per ton) of feed.

14. A composition suitable for controlling helmintic parasites in an animal, in the form of an implant comprising:
a biocompatible implant material; and
a helmintic parasite controlling amount of the peptide FK-565 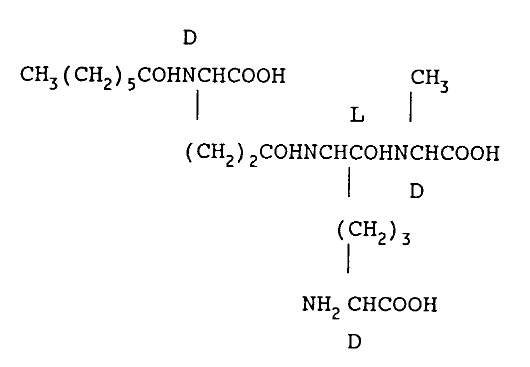 used in admixture with said implant material.

15. The composition of claim 14, wherein said implant is designed to deliver from 0.001 to 5 mg/kg body weight/day.

## Patentansprüche

1. Verwendung des Peptids FK-565 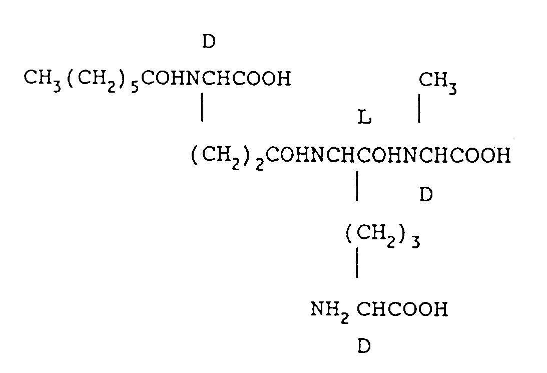 zur Herstellung eines Mittels zur Verwendung bei der Behandlung und/oder bei der Kontrolle von Infektionen durch Helminthparasiten bei Tier oder Mensch.

2. Verwendung nach Anspruch 1, worin die Helminthparasiten ausgewählt sind unter Trematoden, Cestoden und Nematoden.

3. Verwendung nach Anspruch 1 oder Anspruch 2, worin die Helminthparasiten ausgewählt sind unter Helminthparasiten, die den Respirations- und Gastrointestinaltrakt des Tiers infizieren.

4. Verwendung nach Anspruch 1, worin die Helminthparasiten ausgewählt sind unter Saugwürmern, Lungenwürmern, Fadenwürmern, Bandwürmern, Springwürmern und Hakenwürmern.

5. Verwendung nach einem der Ansprüche 1 bis 4, worin die Tiere ausgewählt sind unter bovinen, porcinen, caninen, felinen, equinen, avianen und ovinen Tieren.

6. Verwendung nach Anspruch 5, worin die Tiere ausgewählt sind unter Rind, Schwein, Schaf, Huhn und Truthahn.

7. Verwendung nach einem der Ansprüche 1 bis 6, worin die Zusammensetzung das Peptid FK-565 in einer Menge umfaßt, die ausreicht, um dem Tier oder Menschen 0,001 bis 5 mg/kg Körpergewicht Zuzuführen.

8. Verwendung nach einem der Ansprüche 1 bis 7 zur Herstellung einer Zusammensetzung in Form einer injizierbaren Formulierung, die zur Kontrolle von Helminthparasiten in Tier oder Mensch geeignet ist.

9. Verwendung nach Anspruch 8, worin die injizierbare Formulierung ein Vehikel umfaßt, das ausgewählt ist unter Squalen, Carboxymethylcellulose (CMC), physiologischer Kochsalzlösung oder Hank's Balanced Salzlösung.

10. Verwendung nach einem der Ansprüche 1 bis 7 zur Herstellung einer Zusammensetzung in einer zur oralen Verabreichung geeigneten Form.

11. Verwendung nach Anspruch 10 zur Herstellung einer Zusammensetzung in Form von Tabletten, Suspensionen, Lösungen, Boli, Emulsionen, Kapseln, Pulver, Sirup, Trinkwasserzusammensetzungen und Futterzusammensetzungen.

12. Futterzusammensetzung zur oralen Verabreichung, die zur Kontrolle von Helminthparasiten bei Tier oder Mensch geeignet ist, in Form einer Futterzusammensetzung, umfassend:
* ein ernährungsmäßig ausgewogenes Futter; und
* eine Helminthparasiten-kontrollierende Menge des Peptids FK-565 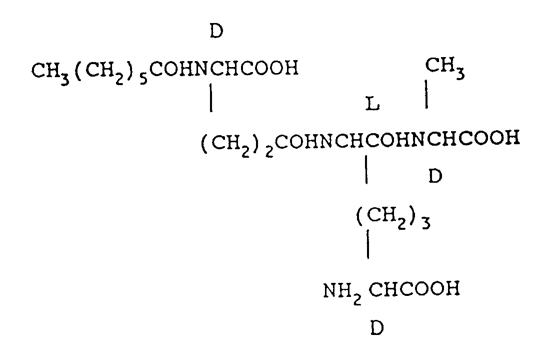 im Gemisch mit dem Futter.

13. Futterzusammensetzung nach Anspruch 12, worin FK-565 zugesetzt ist zu oder verwendet wird im Gemisch mit der Futterzusammensetzung in einer Menge von 11 bis 110 mg/kg (10 bis 100 g pro Tonne) Futter.

14. Zusammensetzung, geeignet zur Kontrolle von Helminthparasiten im Tier, in Form eines Implantates, umfassend:
* ein biokompatibles Implantatmaterial; und
* eine Helminthparasiten-kontrollierende Menge des Peptids FK-565 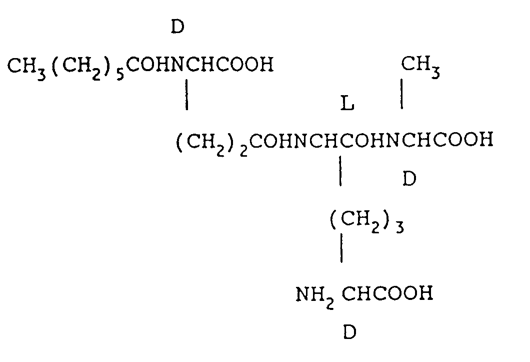
verwendet im Gemisch mit dem Implantatmaterial.

15. Zusammensetzung nach Anspruch 14, worin das Implantat so ausgelegt ist, daß es 0,001 bis 5 mg/kg Körpergewicht/Tag abgibt.

## Revendications

1. Utilisation du peptide FK-565 : 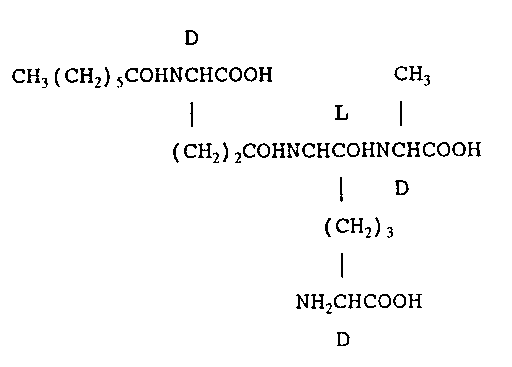 en vue de la préparation d'une composition à utiliser en vue de traiter et/ou de combattre des infections à parasites helminthiques chez l'animal ou l'être humain.

2. Utilisation suivant la revendication 1, caractérisée en ce que lesdits parasites helminthiques sont choisis parmi les trématodes, les cestodes et les nématodes.

3. Utilisation suivant la revendication 1 ou la revendication 2, caractérisée en ce que les parasites helminthiques précités sont choisis parmi les parasites helminthiques qui infectent les voies respiratoires et gastro-intestinales de l'animal précité.

4. Utilisation suivant la revendication 1, caractérisée en ce que lesdits parasites helminthiques sont choisis parmi les digènes, les strongyloïdés, les ascaris, les taenidae, les oxyures et les ankylostomes.

5. Utilisation suivant l'une quelconque des revendications 1 à 4, caractérisée en ce que les animaux précités sont choisis parmi les bovins, les porcins, les canidés, les félins, les équidés, les ovins et les oiseaux.

6. Utilisation suivant la revendication 5, caractérisée en ce que les animaux sont choisis parmi le bétail, les porcs , les moutons, les poulets et les dindons.

7. Utilisation suivant l'une quelconque des revendications 1 à 6, caractérisée en ce que ladite composition comprend le peptide FK-565 en une proportion qui suffit à administrer de 0,001 à 5 mg/kg de poids du corps à l'animal ou l'être humain précité.

8. Utilisation suivant l'une quelconque des revendications 1 à 7, en vue de la confection d'une composition qui se présente sous la forme d'une préparation injectable convenant pour combattre des parasites helminthiques chez les animaux ou les êtres humains.

9. Utilisation suivant la revendication 8, caractérisée en ce que ladite préparation injectable comprend un véhicule choisi parmi le squalène, la carboxyméthylcellulose (CMC), une solution saline physiologique et une solution de sel équilibrée de Hank.

10. Utilisation suivant l'une quelconque des revendications 1 à 7, pour la préparation d'une composition qui se présente sous une forme convenant à l'administration par la voie orale.

11. Utilisation suivant la revendication 10, en vue de la préparation d'une composition qui se présente sous la forme de comprimés, de suspensions, de solutions, de bols, d'émulsions, de gélules, de poudres, de sirops, de compositions d'eaux de boisson et de compositions alimentaires.

12. Composition alimentaire destinée à l'administration par la voie orale, convenant pour combattre des parasites helminthiques chez l'animal ou l'être humain, qui se présente sous une forme de composition alimentaire et comprenant :
un aliment nutritionnellement équilibré, et
une proportion permettant de combattre les parasites helminthiques du peptide FK-565 : 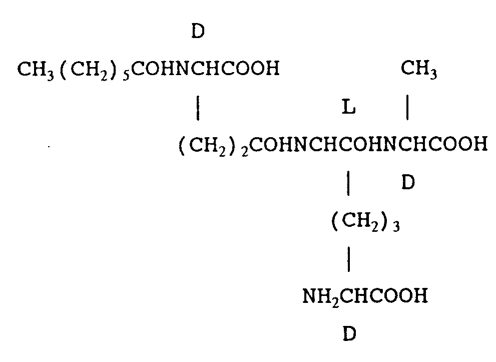 en mélange à l'aliment précité.

13. Composition alimentaire suivant la revendication 12, caractérisée en ce que l'on applique ledit peptide FK-565 à, ou on l'utilise en mélange à ladite composition alimentaire en proportions de 11 à 110 mg/kg (10 à 100 g/tonne) d'aliment.

14. Composition convenant pour combattre des parasites helminthiques chez un animal, caractérisée en ce qu'elle se présente sous la forme d'un implant comprenant :
une matière pour implant biocompatible, et
une proportion permettant de combattre les parasites helminthiques du peptide FK-565 : 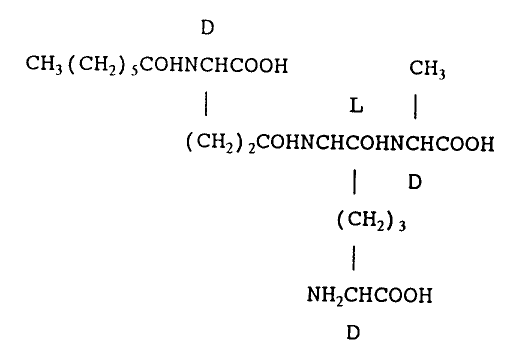 utilisée en mélange à la matière pour implant précitée.

15. Composition suivant la revendication 14, caractérisée en ce que l'implant précité est destiné à débiter de 0,001 à 5 mg/kg de poids corporel/jour.
